# FASCICULE DE BREVET EUROPEEN

(11) **EP 1 357 894 B1**
(45) Date de publication et mention de la délivrance du brevet: **13.04.2011**
(21) Numéro de dépôt: 02702452.0
(22) Date de dépôt: 05.02.2002
(51) Int. Cl.: C11D 1/66, A61K 8/06, A61K 8/60, A61Q 19/00

(54) **UTILISATION D'ALKYLPOLYXYLOSIDES COMME AGENTS EMULSIONNANTS POUR LA PREPARATION D'EMULSIONS EAU-DANS-HUILE.**
VERWENDUNG VON ALKYLPOLYXYLOSIDEN ALS EMULGATOREN ZUR HERSTELLUNG VON W/O-EMULSIONEN
USE OF ALKYLPOLYXYLOSIDES AS EMULSIFYING AGENTS FOR PREPARING WATER-IN-OIL EMULSIONS

(30) Priorité: 05.02.2001 FR 0101480; 05.10.2001 FR 0112878
(43) Date de publication de la demande: 05.11.2003
(73) Titulaire: S.E.P.P.I.C., SOCIETE D'EXPLOITATION DE PRODUITS POUR LES INDUSTRIES CHIMIQUES, 75007 Paris (FR)
(72) Inventeur: AMALRIC, Chantal, F-81700 BLAN (FR); ROSO, Alicia, F-81710 SAIX (FR); MICHEL, Nelly, F-94700 MAISONS ALFORT (FR); TABACCHI, Guy, F-75007 Paris (FR); MILIUS, Alain, F-06000 NICE (FR)
(74) Mandataire: Conan, Philippe Claude
(86) Numéro de dépôt international: PCT/FR2002/000431
(87) Numéro de publication internationale: WO 2002/062313

(56) Documents cités:
- EP-A- 0 507 047
- EP-A- 1 142 901
- DE-A- 4 311 159
- FR-A- 2 756 195
- US-A- 5 681 949
- US-H- H 171
- L. BERTHELOT ET AL: "Behavior of amphiphilic neoglycolipids at the air/solution interface. Interaction with a specific lectin" COLLOIDS AND SURFACES B:BIOINTERFACES, vol. 11, no. 5, 1998, pages 239-248, XP001059815
- PATENT ABSTRACTS OF JAPAN vol. 2000, no. 13, 5 février 2001 (2001-02-05) & JP 2000 302666 A (KAO CORP), 31 octobre 2000 (2000-10-31)

## Description

La présente invention a pour objet l'utilisation d'alkylpolyxylosides particuliers comme agents émulsionnants pour la préparation d'émulsions eau-dans-huile, ainsi que les émulsions eau-dans-huile en contenant.

L'invention trouve application notamment dans le domaine cosmétique.

La demande de brevet FR 00 04414, déposée le 6 avril 2000, décrit des composés de formule :

R-O-(X)ₚ

dans laquelle :
p représente un nombre décimal compris entre 1 et 5,
X représente le reste du xylose, et
R représente un radical alkyle ramifié de formule :

   CH(CₙH₂ₙ₊₁)(CₘH₂ₘ₊₁)-CH₂-

   dans laquelle m est un nombre entier compris entre 6 et 18, n est un nombre entier compris entre 4 et 18 et la somme n + m est supérieure ou égale à 10.

Ces composés sont utiles comme agents tensioactifs.

Des exemples d'alkylpolyglucosides, obtenus à partir d'un alcool gras, sont par ailleurs décrits dans la demande de brevet FR-A-2 790 977.

Il ressort de ces exemples que seuls les alkylpolyglucosides obtenus à partir d'alcool oléique et/ou isostéarylique, permettent d'obtenir une émulsion de type eau-dans-huile. Les autres alkylpolyglucosides, notamment ceux obtenus à partir d'alcool isooctadécylique et d'alcool de Guerbet possédant 18 atomes de carbone, conduisent à des émulsions de type huile-dans-eau.

Il a maintenant été découvert contre toute attente que des alkylpolyxylosides obtenus à partir d'un alcool de Guerbet ayant de 16 à 28 atomes de carbone, permettent d'obtenir des émulsions eau-dans-huile stables.

Ainsi, selon un premier aspect, l'invention a pour objet l'utilisation d'un alkylpolyxyloside de formule :

R-O-(X)_{P} (I)

dans laquelle :
p représente un nombre décimal compris entre 1 et 5,
X représente le reste du xylose, et
R représente un radical alkyle ramifié de formule :

   CH(CₙH₂ₙ₊₁)(CₘH₂ₘ₊₁)-CH₂-

   dans laquelle m est un nombre entier compris entre 6 et 12, n est un nombre entier compris entre 8 et 14 et la somme n + m est comprise dans la gamme de 14 à 26 ;
   ou bien d'une composition consistant en un mélange d'au moins deux composés tels que définis ci-dessus ;
   comme agent émulsionnant pour la préparation d'émulsions eau-dans-huife.

De préférence, la somme n + m est égale à 14, 16, 18, 22 ou 26 et R représente plus particulièrement un radical 2-hexyldécyle (m = 6, n = 8), 2-octyldécyle (m = 8, n = 8), 2-hexyldodécyle (m = 6, n = 10), 2-octyldodécyle (m = 8, n = 10), 2-décyltétradécyle (m = 10, n = 12) ou 2-dodécylhexadécyle (m = 12, n = 14). De manière particulièrement préférée, la somme m + n est égale à 18, 22 ou 26. Et encore de préférence la somme m + n est égale à 22 ou 26.

Dans la formule R-O-(X)_{P}, le groupe R-O- est lié à X par le carbone anomérique du reste de xylose, de manière à former une fonction acétal.

p, qui représente le degré moyen de polymérisation du xylose, est plus particulièrement compris entre 1 et 2,5 et tout particulièrement entre 1 et 2,0.

Le composé de formule R-O-(X)_{P} peut être préparé en faisant réagir le xylose avec un excès d'alcool gras de formule ROH, puis élimination de l'alcool gras n'ayant pas réagi.

Dans le procédé tel que défini ci-dessus, la réaction est effectuée en présence de catalyseurs acides forts.

Selon une variante du procédé tel que défini précédemment, le xylose est mis à réagir avec un alcool de formule R₁-OH, dans laquelle R₁ comporte de 1 à 4 atomes de carbone et plus particulièrement, avec le butanol, pour conduire à l'acétal de formule R₁O-(X)ₚ, qui subit ensuite une trans-acétalisation par un excès d'alcool de formule ROH avec distillation de l'alcool de formule R₁OH formé puis élimination de l'alcool de formule ROH n'ayant pas réagi.

Dans ce procédé et sa variante, tels que décrits précédemment, l'élimination de l'alcool de formule ROH n'ayant pas réagi, est effectuée selon des méthodes connues de l'homme du métier comme, par exemple, la distillation, la distillation sur film mince, la distillation moléculaire ou l'extraction par solvants, ou par fluides supercritiques.

Selon un second aspect, l'invention a pour objet une émulsion eau-dans-huile comprenant de 0,2 à 25 % en poids, de préférence de 0,2 à 10 % en poids, et de préférence encore de 0,5 à 5 % en poids, d'un ou plusieurs alkylpolyxylosides tels que définis ci-dessus.

Selon un mode de réalisation avantageux, chaque alkylpolyxyloside de formule (I) est en mélange avec son alcool de Guerbet correspondant (de formule ROH où R a la signification mentionnée précédemment), dans un rapport pondéral alkylpolyxyloside/alcool compris dans la gamme de 1/99 à 99/1.

L'émulsion eau-dans-huile comprend généralement de 5 à 90 % en poids, de préférence de 10 à 70 % en poids, d'une ou plusieurs huiles choisies notamment parmi :
- les huiles d'origine végétale, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ;
- les huiles végétales et leurs esters méthyliques éthoxylés ;
- les huiles d'origine animale, telles que le squalène, le squalane ;
- les huiles minérales, telles que l'huile de paraffine, l'huile de vaseline et les isoparaffines ;
- les huiles synthétiques, notamment les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane, l'isododécane, les huiles perfluorées et les huiles de silicone. Parmi ces dernières, on peut plus particulièrement citer les diméthylpolysiloxanes, méthylphénylpolysiloxanes, les silicones modifiés par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiés par des groupements fluorés, des silicones cycliques et des silicones modifiés par des groupements alkyles.

Cette huile peut également être choisie parmi les acides gras, les alcools gras, les cires d'origine naturelle ou synthétique, et plus généralement encore tout corps gras d'origine végétale, animale ou synthétique.

L'émulsion eau-dans-huile conforme à la présente invention peut également contenir, de façon optionnelle, jusqu'à 10 % en poids d'un co-émulsionnant et jusqu'à 10 % en poids d'un stabilisant.

Parmi les agents stabilisants susceptibles d'être utilisés dans le cadre de la présente invention, on peut citer l'huile de ricin hydrogénée ; les cires végétales ou animales comme par exemple la cire d'abeille et la cire de carnauba ; l'acide stéarique et ses sels métalliques comme le stéarate d'aluminium ; les silices hydrophobes ; les copolymères polyéthylèneglycol-alkylglycol comme le PEG-45 dodécylglycol copolymère tel que le produit commercialisé sous la dénomination ELFACOS ST 9® ; les polymères tels que les produits commercialisés sous la dénomination KRATON® ; les cires minérales comme l'ozokérite ; les argiles comme l'hectorite ou la bentonite ; les amidons modifiés hydrophobes comme par exemple le produit commercialisé sous la dénomination DRY FLOW PC ®.

Parmi les agents co-émulsionnants susceptibles d'être utilisés dans le cadre de la présente invention, on citera notamment les lipoaminoacides et leurs sels, les lipopeptides et leurs sels, les émulsionnants siliconés non-ioniques et anioniques, les esters de sorbitan, les esters de polyglycérol, l'huile de ricin hydrogénée éthoxylée, le stéarate de glycérol, les polyhydroxystéarates de polyol comme par exemple le produit dénommé HYPERMER® B241 ; les émulsionnants cationiques comme par exemple les aminoxydes, le quaternium 82 ; les esters de sucrose, les esters de méthylglucoside ethoxylés ou non ; les acides gras éthoxylés ; les alcools gras éthoxylés ; les émulsionnants anioniques comme le décylphosphate ou le cétéarylsulfate.

L'émulsion eau-dans-huile comprend aussi avantageusement un ou plusieurs sel minéraux, comme par exemple le chlorure de magnésium, le sulfate de magnésium, le borate de sodium ou le chlorure de sodium, en une quantité allant de 0,1 % à 5 % en poids.

Les émulsions eau-dans-huile conformes à la présente invention, peuvent être préparées par simple dispersion, à une température comprise entre 15°C et 90°C, de la phase aqueuse dans la phase huileuse, en présence du ou des émulsionnants, et éventuellement du ou des stabilisants.

D'une façon connue en soi, ces émulsions peuvent en outre comprendre un ou plusieurs composés choisis parmi les humectants, comme par exemple la glycérine, les conservateurs comme par exemple les produits connus sous la dénomination SEPICIDE®, les colorants, les parfums, les actifs cosmétiques, les filtres solaires minéraux ou organiques, les charges minérales comme les oxydes de fer, oxydes de titane et le talc, les charges synthétiques comme les nylons et les poly(méthacrylate de méthyle) réticulés ou non, les élastomères silicone, les séricites et les extraits de plantes.

Ces composés pourront être introduits dans la phase aqueuse ou dans la phase huileuse, selon leur affinité pour ces phases, soit au cours de la phase de dispersion précitée, soit en ce qui concerne les composés sensibles à la température, postérieurement au cours de la phase de refroidissement dans le cas où la dispersion est réalisée à chaud.

L'invention sera illustrée par les exemples suivants.

### Exemple 1 : Préparation d'un 2-octyldécylxyloside et 2-hexyldodécylxyloside

On chauffe à 90°C, dans un réacteur, un mélange d'alcools comprenant majoritairement du 2-hexyl dodécanol et du 2-octyl décanol, commercialisé sous le nom Isofol®18 par la Société SASOL, puis on ajoute sous agitation du xylose dans le rapport stoechiométrique xylose / alcools = 1/6 et laisse réagir pendant 4 heures en présence d'un catalyseur acide. Après refroidissement, neutralisation et filtration, on obtient un produit présentant un indice d'hydroxyle de 241 et contenant 15 % en poids d'un mélange de 2-octyldécylxyloside et de 2-hexyldodécylxyloside, et 85 % en poids d'un mélange de 2-octyldécanol et de 2-hexyldodécanol.

### Exemple 2: Préparation d'un 2-octyldécylxyloside et 2-hexyldodécylxyloside

Le produit obtenu dans l'exemple 1 est distillé partiellement pour conduire à un produit contenant 60 % en poids d'un mélange de 2-octyldécylxyloside et de 2-hexyldodécylxyloside, et 40% en poids d'un mélange de 2-octyldécanol et de 2-hexyldodécanol.

### Exemple 3: Préparation d'un 2-octyldécylxyloside et 2-hexyldodécylxyloside

Le produit obtenu dans l'exemple 1 est distillé totalement pour conduire à 100% d'un mélange de 2-octyldécylxyloside et de 2-hexyldodécylxyloside.

### Exemple 4 : Préparation d'un 2-décyltétradécylxyloside

61,8 kg de 2-décyltétradécanol, commercialisé par la Société SASOL sous l'appellation Isofol®24, sont introduits dans un réacteur. 8,7 kg de xylose sont dispersés progressivement dans le milieu agité et 65 g d'acide sulfurique sont alors ajoutés. Le mélange est maintenu à 115°C pendant 6 heures, sous vide partiel, puis neutralisé par la lessive de soude. Après filtration, le liquide limpide obtenu présente un indice d'hydroxyle de 183 et contient 15% en poids de 2-décyltétradécylxyloside et 85% en poids de 2-décyltétradécanol.

### Exemple 5 : Préparation d'un 2-dodécylhexadécylxyloside

Dans les mêmes conditions stoechiométriques et réactionnelles que l'exemple 4, on fait réagir du 2-dodécylhexadécanol, commercialisé par la Société SASOL sous l'appellation Isofol®28, avec du xylose pour conduire à un liquide présentant un indice d'hydroxyle de 141 et contenant 15% en poids de 2-dodécylhexadécylxyloside et 85% en poids de 2-dodécylhexadécanol.

### Exemple comparatif 1 : Préparation d'un isostéarylglucoside

On introduit dans un réacteur de l'alcool isostéarylique (produit commercialisé par la Société UNIQEMA sous la dénomination PRISORlNE®3515, ou par la Société COGNIS sous la dénomination SPEZIOL®C18 ISO).

On introduit également dans le réacteur du glucose, de sorte que le rapport molaire entre l'alcool isostéarylique et le glucose soit de : 6/1.

On fait ensuite réagir le glucose avec l'alcool gras pendant 6 heures à une température d'environ 100°C, en présence d'un catalyseur acide sous vide partiel.

Après réaction, on neutralise le catalyseur au moyen d'une base.

Le produit obtenu contient 15 % en poids d'isostéarylglucoside et 85 % en poids d'alcool isostéarylique.

### Exemple comparatif 2: Préparation d'un isostéarylxyloside

On procède comme dans l'exemple comparatif 1 en remplaçant le glucose par du xylose.

Le produit obtenu contient 15% en poids d'isostéarylxyloside et 85% en poids d'alcool isostéarylique

### Exemple comparatif 3: Préparation d'un 2-octyldécylglucoside et 2-hexyfdodécylglucoside

On procède comme dans l'exemple 1 en remplaçant le xylose par du glucose.

Le produit obtenu contient 15% en poids d'un mélange de 2-octyldécylglucoside et de 2-hexyldodécylglucoside et 85% en poids d'un mélange de 2-octyldécanol et de 2-hexyldodécanol.

### Exemple 6 : Mise en évidence des propriétés émulsionnantes sur l'huile de paraffine

On prépare les émulsions de la manière suivante :
La phase aqueuse est chauffée à une température de 70 à 85°C. Parallèlement la phase grasse contenant le système émulsionnant de l'invention et les huiles est chauffée à une température identique de 70 à 85°C.

Les 2 phases sont ensuite mélangées et émulsionnées à l'aide d'un émulseur rotor stator (par exemple un mélangeur de laboratoire de marque SILVERSON pendant 4 min. à 4000 tr/min.). Après émulsification, l'émulsion est refroidie sous agitation modérée.

Toutes les émulsions obtenues sont du type eau-dans huile.

Les résultats sont présentés dans le Tableau 1.

Ces résultats illustrent :
- l'absence de pouvoir émulsionnant pour l'alkylpolyglucoside obtenu à partir d'un alcool de Guerbet ;
- la meilleure stabilité des émulsions selon l'invention, obtenues avec des alkylpolyxylosides sur alcool de Guerbet par rapport aux alkylpolyglucosides et alkylpolyxylosides dérivés d'alcool isostéarylique.

### Exemple 7 : Mise en évidence des propriétés émulsionnantes sur un C8-C10 triglycéride

Les émulsions sont préparées selon le mode opératoire de l'exemple 6. Toutes les émulsions obtenues sont de type eau-dans-huile

Les résultats sont présentés dans le Tableau 2.

Là encore les résultats illustrent :
- l'absence de performance pour l'alkylpolyglucoside obtenu à partir d'un alcool de Guerbet ;
- la performance supérieure des alkylpolyxylosides selon l'invention par rapport aux émulsionnants des exemples comparatifs 1 et 2.

### Exemple 8 : Mise en évidence des propriétés émulsionnantes sur le squalane

Les émulsions sont préparées selon le mode opératoire de l'exemple 6. Toutes les émulsions obtenues sont du type eau-dans-huile.

Les résultats sont présentés dans le Tableau 3.

Là encore les résultats illustrent :
- l'absence de performance pour l'alkylpolyglucoside obtenu à partir d'un alcool de Guerbet ;
- la performance supérieure pour des alkylpolyxylosides selon l'invention par rapport aux émulsionnants des exemples comparatifs 1 et 2.

### Exemple 9 : Mise en évidence des propriétés émulsionnantes sur le cétéaryloctanoate

Les émulsions sont préparées selon le mode opératoire de l'exemple 6. Toutes les émulsions obtenues sont du type eau-dans-huile.

Les résultats sont présentés dans le Tableau 4.

Là encore les résultats illustrent :
- l'absence de performance pour l'alkylpolyglucoside obtenu à partir d'un alcool de Guerbet ;
- la performance supérieure des alkylpolyxylosides selon l'invention par rapport aux émulsionnants des exemples comparatifs 1 et 2.

### Exemple 10 : Mise en évidence des propriétés émulsionnantes en association avec d'autres émulsionnants

Les émulsions sont préparées de la manière suivante.

Les 2 phases sont mélangées sans chauffage préalable et émulsionnées à l'aide d'un émulseur rotor stator (de type mélangeur de laboratoire SILVERSON pendant 4 min. à 4000 tr/min.).

Toutes les émulsions obtenues sont du type eau-dans-huile.

Les résultats sont présentés dans le Tableau 5.

Même en association avec d'autres émulsionnants lipophiles, la performance des alkylpolyxylosides selon l'invention est supérieure à celle des émulsionnants des exemples comparatifs 1 et 2.

**Tableau 1**

| | Emulsion avec ex 1 | Emulsion avec ex 2 | Emulsion avec ex 3 | Emulsion avec ex 4 | Emulsion avec ex 5 | Emulsion avec ex comp. 1 | Emulsion avec ex comp. 2 | Emulsion avec ex comp. 3 |
|---|---|---|---|---|---|---|---|---|
| 2-octyldécylxyloside et 2-hexyldodécylxyloside | 1,2 | 1,2 | 1,2 | | | | | |
| 2-décyltétradécylxyloside | | | | 1,2 | | | | |
| 2-dodécylhexadécylxyloside | | | | | 1,2 | | | |
| Isostéarylglucoside | | | | | | 1,2 | | |
| Isostéarylxyloside | | | | | | | 1,2 | |
| 2-octyldécylglucoside et 2-hexyldodécylglucoside | | | | | | | | 1,2 |
| 2-octyldécanol et 2-hexyldodécanol | 6,8 | 1,8 | | | | | | 6,8 |
| Décyltétradécanol | | | | 6,8 | | | | |
| Dodécylhexadécanol | | | | | 6,8 | | | |
| Alcool isostéarylique | | | | | | 6,8 | 6,8 | |
| PEG 45 dodécylglycol copolymère | 2 | 2 | 2 | 2 | 2 | 2 | 2 | 2 |
| Huile de paraffine | 40 | 45 | 46,8 | 40 | 40 | 40 | 40 | 40 |
| Glycérine | 5 | 5 | 5 | 5 | 5 | 5 | 5 | 5 |
| Eau | 44,3 | 44,3 | 44,3 | 44,3 | 44,3 | 44,3 | 44,3 | 44,3 |
| MgSO₄, 7H₂O | 0,7 | 0,7 | 0,7 | 0,7 | 0,7 | 0,7 | 0,7 | 0,7 |
| | | | | | | | | |
| Stabilité | Exsudation huileuse à 6 mois | Exsudation huileuse à 6 mois | Exsudation huileuse à 6 mois | > 6 mois | > 6 mois | Exsudation huileuse à 1 mois | Exsudation huileuse 3 mois | Pas à d'émulsification |

**Tableau 2**

| | Emulsion avec ex 1 | Emulsion avec ex 4 | Emulsion avec ex comp. 1 | Emulsion avec ex comp. 2 | Emulsion avec ex comp. 3 |
|---|---|---|---|---|---|
| 2-octyldécylxyloside et 2-hexyldodécylxyloside | 1,2 | | | | |
| 2-décyltétradécylxyloside | | 1,2 | | | |
| Isostéarylglucoside | | | 1,2 | | |
| Isostéarylxyloside | | | | 1,2 | |
| 2-octyldécylglucoside et 2-hexyldodécylglucoside | | | | | 1,2 |
| 2-octyldécanol et 2-hexyldodécanol | 6,8 | | | | 6,8 |
| 2-décyltétradécanol | | 6,8 | | | |
| Alcool isostéarylique | | | 6,8 | 6,8 | |
| PEG 45 dodécylglycol copolymère | 2 | 2 | 2 | 2 | 2 |
| C8-C10 triglycéride | 40 | 40 | 40 | 40 | 40 |
| Glycérine | 5 | 5 | 5 | 5 | 5 |
| Eau | 44,3 | 44,3 | 44,3 | 44,3 | 44,3 |
| MgSO₄, 7H₂O | 0,7 | 0,7 | 0,7 | 0,7 | 0,7 |
| | | | | | |
| Stabilité | > 6 mois | > 6 mois | Pas d'émulsification | Exsudation huileuse à 7 jours | Pas d'émulsification |

**Tableau 3**

| | Emulsion avec ex 1 | Emulsion avec ex 4 | Emulsion avec excomp.1 1 | Emulsion avec ex comp. 2 | Emulsion avec ex comp. 3 |
|---|---|---|---|---|---|
| 2-octyldécylxyloside et 2-hexyldodécylxyloside | 1,2 | | | | |
| 2-décyltétradécylxyloside | | 1,2 | | | |
| Isostéarylglucoside | | | 1,2 | | |
| Isostéarylxyloside | | | | 1,2 | |
| 2-octyldécylglucoside et 2-hexyldodécylglucoside | | | | | 1,2 |
| et 2-hexyldodécanol | 6,8 | | | | 6,8 |
| 2-octyldécanol Décyltétradécanol | | 6,8 | | | |
| Alcool isostéarylique | | | 6,8 | 6,8 | |
| PEG 45 dodécylglycol copolymère | 2 | 2 | 2 | 2 | 2 |
| Squalane | 40 | 40 | 40 | 40 | 40 |
| Glycérine | 5 | 5 | 5 | 5 | 5 |
| Eau | 44,3 | 44,3 | 44,3 | 44,3 | 44,3 |
| MgSO₄, 7H₂O | 0,7 | 0,7 | 0,7 | 0,7 | 0,7 |
| | | | | | |
| Stabilité | Exsudation huileuse à 6 mois | > 6 mois | Exsudation huileuse à 1 mois | Exsudation huileuse à 1 mois | Pas d'émulsification |

**Tableau 4**

| | Emulsion avec ex 1 | Emulsion avec ex 4 | Emulsion avec ex comp. 1 | Emulsion avec ex comp. 2 | Emulsion avec ex comp. 3 |
|---|---|---|---|---|---|
| 2-octyldécylxyloside et 2-hexyldodécylxyloside | 1,2 | | | | |
| 2-décyltétradécylxyloside | | 1,2 | | | |
| Isostéarylglucoside | | | 1,2 | | |
| Isostéarylxyloside | | | | 1,2 | |
| 2-octyldécylglucoside et 2-hexyldodécylglucoside | | | | | 1,2 |
| 2-octyldécanol et 2-hexyldodécanol | 6,8 | | | | 6,8 |
| Décyltétradécanol | | 6,8 | | | |
| Alcool isostéarylique | | | 6,8 | 6,8 | |
| PEG 45 dodécylglycol copolymère | 2 | 2 | 2 | 2 | 2 |
| Cétéaryloctanoate | 40 | 40 | 40 | 40 | 40 |
| Glycérine | 5 | 5 | 5 | 5 | 5 |
| Eau | 44,3 | 44,3 | 44,3 | 44,3 | 44,3 |
| MgSO₄, 7H₂O | 0,7 | 0,7 | 0,7 | 0,7 | 0,7 |
| | | | | | |
| Stabilité | Exsudation huileuse à 6 mois | > 6 mois | Pas d'émulsification | Exsudation huileuse à 3 mois | Pas d'émulsification |

**Tableau 5**

| | Emulsion avec ex 4 | Emulsion avec ex comp. 1 | Emulsion avec ex comp. 2 | Emulsion avec ex 4 | Emulsion avec ex comp. 1 | Emulsion avec ex comp. 2 |
|---|---|---|---|---|---|---|
| 2-décyltétradécylxyloside | 0,9 | | | 0,9 | | |
| Isostéarylglucoside | | 0,9 | | | 0,9 | |
| Isostéarylxyloside | | | 0,9 | | | 0,9 |
| 2-décyltétradécanol | 5,1 | | | 5,1 | | |
| Alcool isostéarylique | | 5,1 | 5,1 | | 5,1 | 5,1 |
| Triglycéride C8-C10 | 40 | 40 | 40 | 40 | 40 | 40 |
| PEG 150 polyhydroxystéarate | 4 | 4 | 4 | | | |
| Diméthicone copolyol | | | | 4 | 4 | 4 |
| Glycérine | 5 | 5 | 5 | 5 | 5 | 5 |
| Eau | 44,3 | 44,3 | 44,3 | 44,3 | 44,3 | 44,3 |
| mgSO₄, 7H₂O | 0,7 | 0,7 | 0,7 | 0,7 | 0,7 | 0,7 |
| | | | | | | |
| Stabilité | > 6 mois | Exsudation huileuse à 7 jours | Exsudation huileuse à 1 mois | > 6 mois | Pas d'émulsification | Exsudation huileuse à 7 jours |

## Revendications

1. Utilisation d'un alkylpolyxyloside ou d'un mélange d'alkylpolyxylosides de formule :
R-O-(X)ₚ (I)
dans laquelle :
p représente un nombre décimal compris entre 1 et 5,
X représente le reste du xylose, et
R représente un radical alkyle ramifié de formule :
CH(CₙH₂ₙ₊₁)(CₘH₂ₘ₊₁)-CH₂-
dans laquelle m est un nombre entier compris entre 6 et 12, n est un nombre entier compris entre 8 et 14 et la somme n + m est comprise dans la gamme de 14 à 26 ;
comme agent émulsionnant pour la préparation d'émulsions eau-dans-huile.

2. Utilisation selon la revendication 1, dans laquelle dans la formule (I) la somme m + n est égale à 14, 16, 18, 22 ou 26, de préférence égale à 18, 22 ou 26, de préférence encore égale à 22 ou 26.

3. Utilisation selon la revendication 1 ou 2, dans laquelle dans la formule (I) R représente un radical 2-hexyldécyle, 2-octyldécyle, 2-hexyldodécyle, 2-octyldodécyle, 2-décyltétradécyle ou 2-dodécylhexadécyle.

4. Emulsion eau-dans-huile, qui comprend de 0,2 à 25 % en poids, de préférence de 0,2 à 10 % en poids, et de préférence encore de 0,5 à 5 % en poids, d'un alkylpolyxyloside ou d'un mélange d'alkylpolyxylosides de formule :
R-O-(X)ₚ (I)
dans laquelle :
p représente un nombre décimal compris entre 1 et 5,
X représente le reste du xylose, et
R représente un radical alkyle ramifié de formule :
CH(CₙH₂ₙ₊₁)(CₘH₂ₘ₊₁)-CH₂-
dans laquelle m est un nombre entier compris entre 6 et 12, n est un nombre entier compris entre 8 et 14 et la somme n + m est comprise dans la gamme de 14 à 26.

5. Emulsion selon la revendication 4, dans laquelle chaque alkylpolyxyloside est en mélange avec son alcool correspondant de formule ROH, dans un rapport -pondéral alkylpolyxyloside/alcool compris dans la gamme de 1/99 à 99/1.

6. Emulsion selon la revendication 4 ou 5, qui comprend de 5 à 90 % en poids, de préférence de 10 à 70 % en poids, d'une ou plusieurs huiles.

7. Emulsion selon l'une des revendications 4 à 6, dans laquelle dans la formule (I) la somme m + n est égale à 14, 16, 18, 22 ou 26, de préférence égale à 18, 22 ou 26, de préférence encore égale à 22 ou 26.

8. Emulsion selon l'une des revendications 4 à 7, dans laquelle dans la formule (I) R représente un radical 2-hexyldécyle, 2-octyldécyle, 2-hexyldodécyle, 2-octyldodécyle, 2-décyltétradécyle ou 2-dodécylhexadécyle.

## Claims

1. Use of an alkylpolyxyloside or of a mixture of alkylpolyxylosides of formula:
R-O-(X)ₚ (I)
in which:
p represents a decimal number between 1 and 5,
X represents the xylose residue, and
R represents a branched alkyl radical of formula:
CH (CₙH₂ₙ₊₁) (CₘH₂ₘ₊₁)-CH₂-
in which m is an integer between 6 and 12, n is an integer between 8 and 14 and the sum n + m is within the range from 14 to 26;
as emulsifying agent in the preparation of water-in-oil emulsions.

2. Use according to Claim 1, in which, in the formula (I), the sum m + n is equal to 14, 16, 18, 22 or 26, preferably equal to 18, 22 or 26, more preferably equal to 22 or 26.

3. Use according to Claim 1 or 2, in which, in the formula (I), R represents a 2-hexyldecyl, 2-octyldecyl, 2-hexyldodecyl, 2-octyldodecyl, 2-decyltetradecyl or 2-dodecylhexadecyl radical.

4. Water-in-oil emulsion, which comprises from 0.2 to 25% by weight, preferably from 0.2 to 10% by weight and more preferably from 0.5 to 5% by weight of an alkylpolyxyloside or of a mixture of alkylpolyxylosides of formula:
R-O-(X)ₚ (I)
in which:
p represents a decimal number between 1 and 5,
X represents the xylose residue, and
R represents a branched alkyl radical of formula:
CH(CₙH₂ₙ₊₁) (CₘH₂ₘ₊₁)-CH₂-
in which m is an integer between 6 and 12, n is an integer between 8 and 14 and the sum n + m is within the range from 14 to 26.

5. Emulsion according to Claim 4, in which each alkylpolyxyloside is present as a mixture with its corresponding alcohol of formula ROH, in an alkylpolyxyloside/alcohol ratio by weight within the range from 1/99 to 99/1.

6. Emulsion according to Claim 4 or 5, which comprises from 5 to 90% by weight, preferably from 10 to 70% by weight, of one or more oils.

7. Emulsion according to one of Claims 4 to 6, in which, in the formula (I), the sum m + n is equal to 14, 16, 18, 22 or 26, preferably equal to 18, 22 or 26, more preferably equal to 22 or 26.

8. Emulsion according to one of Claims 4 to 7, in which, in the formula (I), R represents a 2-hexyldecyl, 2-octyldecyl, 2-hexyldodecyl, 2-octyldodecyl, 2-decyltetradecyl or 2-dodecylhexadecyl radical.

## Patentansprüche

1. Verwendung eines Alkylpolyxylosids oder eines Gemischs von Alkylpolyxylosiden der Formel:
R-O-(X)ₚ (I)
worin:
p für eine Dezimalzahl zwischen 1 und 5 steht,
X für den Xyloserest steht und
R für einen verzweigten Alkylrest der Formel:
CH(CₙH₂ₙ₊₁) (CₘH₂ₘ₊₁)-CH₂-
worin m eine ganze Zahl zwischen 6 und 12 ist, n eine ganze Zahl zwischen 8 und 14 ist und die Summe n + m im Bereich von 14 bis 26 liegt, steht;
als Emulgator zur Herstellung von Wasser-in-Öl-Emulsionen.

2. Verwendung nach Anspruch 1, wobei in der Formel (I) die Summe m + n gleich 14, 16, 18, 22 oder 26, vorzugsweise gleich 18, 22 oder 26 und noch weiter bevorzugt gleich 22 oder 26 ist.

3. Verwendung nach Anspruch 1 oder 2, wobei in der Formel (I) R für einen 2-Hexyldecyl-, 2-Octyldecyl-, 2-Hexyldodecyl-, 2-Octyldodecyl-, 2-Decyltetradecyl- oder 2-Dodecylhexadecylrest steht.

4. Wasser-in-Öl-Emulsion, die 0,2 bis 25 Gew.-%, vorzugsweise 0,2 bis 10 Gew.-% und noch weiter bevorzugt 0,5 bis 5 Gew.-% eines Alkylpolyxylosids oder eines Gemischs von Alkylpolyxylosiden der Formel:
R-O-(X)_{P} (I)
worin:
p für eine Dezimalzahl zwischen 1 und 5 steht,
X für den Xyloserest steht und
R für einen verzweigten Alkylrest der Formel:
CH(CₙH₂ₙ₊₁) (CₘH₂ₘ₊₁)-CH₂-
worin m eine ganze Zahl zwischen 6 und 12 ist, n eine ganze Zahl zwischen 8 und 14 ist und die Summe n + m im Bereich von 14 bis 26 liegt, steht; enthält.

5. Emulsion nach Anspruch 4, wobei jedes Alkylpolyxylosid im Gemisch mit dem entsprechenden Alkohol der Formel ROH in einem Alkylpolyxylosid/Alkohol-Gewichtsverhältnis im Bereich von 1/99 bis 99/1 vorliegt.

6. Emulsion nach Anspruch 4 oder 5, die 5 bis 90 Gew.-% und vorzugsweise 10 bis 70 Gew.-% eines oder mehrerer Öle enthält.

7. Emulsion nach einem der Ansprüche 4 bis 6, wobei in der Formel (I) die Summe m + n gleich 14, 16, 18, 22 oder 26, vorzugsweise gleich 18, 22 oder 26 und noch weiter bevorzugt gleich 22 oder 26 ist.

8. Emulsion nach einem der Ansprüche 4 bis 7, wobei in der Formel (I) R für einen 2-Hexyldecyl-, 2-Octyldecyl-, 2-Hexyldodecyl-, 2-Octyldodecyl-, 2-Decyltetradecyl- oder 2-Dodecylhexadecylrest steht.
